# EUROPÄISCHE PATENTSCHRIFT

(11) **EP 2 108 181 B1**
(45) Veröffentlichungstag und Bekanntmachung des Hinweises auf die Patenterteilung: **27.05.2015**
(21) Anmeldenummer: 08700842.1
(22) Anmeldetag: 07.01.2008
(51) Int. Cl.: G10K 15/02, A61M 1/14, H04R 1/32

(54) **VERWENDUNG EINER GERICHTETEN SCHALLQUELLE SOWIE MEDIZINISCHER BEHANDLUNGSRAUM**
USE OF A DIRECTED SOUND SOURCE AND MEDICAL TREATMENT ROOM
UTILISATION D'UNE SOURCE ACOUSTIQUE ORIENTÉE ET ESPACE DE TRAITEMENT MÉDICAL

(30) Priorität: 11.01.2007 DE 102007002481
(43) Veröffentlichungstag der Anmeldung: 14.10.2009
(73) Patentinhaber: Fresenius Medical Care Deutschland GmbH, 61352 Bad Homburg (DE)
(72) Erfinder: KLÖFFEL, Peter, 97720 Nüdlingen (DE)
(74) Vertreter: Vièl, Christof
(86) Internationale Anmeldenummer: PCT/DE2008/000005
(87) Internationale Veröffentlichungsnummer: WO 2008/083661

(56) Entgegenhaltungen:
- WO-A-2005/002199
- DE-A1- 3 346 357
- US-A- 5 526 814
- US-A1- 2002 101 360
- US-A1- 2004 124 739
- US-B1- 7 106 180

## Beschreibung

Die Erfindung betrifft die Verwendung einer gerichteten Schallquelle sowie einen medizinischen Behandlungsraum.

Derzeit werden bei Patienten, die sich für längere Zeit an einem bestimmten Behandlungsplatz befinden, akustische Informationen wie Unterhaltung und/oder Informationen übermittelt, indem diese Patienten einen Kopfhörer tragen. Dieser Kopfhörer kann mit einem Gerät verbunden sein, das dem jeweiligen Patienten individuell zugeordnet ist wie beispielsweise ein MP3-Player, ein tragbares CD-Abspielgerät oder ähnliches. Ebenso kann der Kopfhörer einem zentralen Gerät zugeordnet sein, indem beispielsweise mittels Infrarot die Information von dem zentralen Gerät an den entsprechenden Kopfhörer übermittelt wird.

Insbesondere wenn sich mehrere Patienten in einem Raum aufhalten, müssen die Kopfhörer getragen werden, wenn die Patienten nicht alle dasselbe Programm hören wollen. Das Tragen der Kopfhörer wird oftmals als unbequem empfunden.

Zur Lösung dieses Problems wird nach der vorliegenden Erfindung eine Verwendung gemäß Anspruch 1 vorgeschlagen.

Dies kann beispielsweise bei einer Dialyse der Fall sein oder bei einer vergleichsweise langsam einlaufenden Infusion wie beispielsweise bei einer Chemotherapie.

Vorteilhaft müssen die Patienten dann keinen Kopfhörer tragen und können dennoch ohne Störung von Nachbarn oder durch Nachbarn ihr individuelles Programm hören. Dies kann ein reines Audioprogramm sein oder auch der Ton zu einem Film.

Das Fehlen der Kopfhörer ist insofern vorteilhaft, als dies von den Patienten als bequemer empfunden wird und weil weiterhin zu den ohnehin vorhanden Schläuchen und Leitungen, die bei der Behandlung an den Patienten angelegt sind, vorteilhaft die weiteren Leitungen des Kopfhörers entfallen.

Die Unterbringung mehrerer Patienten in einem gemeinsamen größeren Raum hat hinsichtlich der medizinischen Betreuung und Überwachung der Behandlung Vorteile, so dass aus medizinischen Gründen eine akustische und damit auch räumliche Trennung der einzelnen Patienten voneinander nicht machbar ist. Insofern wirkt sich gerade unter diesem Gesichtspunkt die akustische Trennung der Raumeinheiten vorteilhaft aus, in denen beispielsweise die Liegen der einzelnen Patienten stehen.

Es sind bereits gerichtete Schallquellen bekannt. Hierzu ist insbesondere auf die US 2001/0007591 A1 zu verweisen. Dabei wird eine Ultraschallwelle als Trägerwelle verwendet, die zur Übertragung der akustischen Signale entsprechend moduliert wird. Zur Erläuterung dieser Technologie wird ausdrücklich auf diese Veröffentlichung verwiesen.

Derartige Schallquellen können beispielsweise von der Holosonic Research Labs, Inc. bezogen werden.

Aus der US 2004/124739 A1 sind ebenfalls gerichtete Schallquellen bekannt. Dort sind verschiedene Anwendungsgebiete erörtert für die Technologie der gerichteten Schallquellen. Dort sind als Anwendungsgebiete aufgezählt Büro und Haus sowie Unterhaltungsräumlichkeiten. Die Anwendung in medizinischen Behandlungsräumen und insbesondere die Möglichkeit, damit in einem Behandlungsraum mehrere Behandlungsplätze unterbringen zu können, ist dort nicht erwähnt.

Bei der Ausgestaltung nach Anspruch 2 ist der Behandlungsplatz ein Dialysebehandlungsplatz. Es kann sich dabei insbesondere um einen Hämodialysebehandlungsplatz handeln.

Hierbei erweist es sich als vorteilhaft, dass die Patienten während der mehrstündigen Behandlung ihr eigenes, individuelles Programm komfortabel hören können. Diese Behandlung muss abhängig vom Gesundheitszustand des Patienten bis zu drei Mal pro Woche durchgeführt werden.

Bei der Ausgestaltung nach Anspruch 3 wird dem Patienten zugeordnet über die gerichtete Schallquelle eine individualisierte Kategorie von Informationen/Unterhaltung ausgegeben.

Dabei können beispielsweise auf einer Patientenkarte oder in einer Patientendatei neben medizinischen Daten auch bestimmte Hör- bzw. Sehgewohnheiten des Patienten gespeichert sein, so dass beispielsweise ohne eine gesonderte Auswahl des Patienten bei der konkreten Behandlung klassische Musik gespielt wird.

Anspruch 4 betrifft einen medizinischen Behandlungsraum gemäß der Erfindung.

Die Vorteile sind dabei vergleichbar den im Zusammenhang mit Anspruch 1 bereits erörterten Vorteilen.

Bei der Ausgestaltung nach Anspruch 5 ist der medizinische Behandlungsplatz ein Dialysebehandlungsplatz.

Die entsprechenden Vorteile sind im Zusammenhang mit Anspruch 2 bereits erläutert.

Bei der Ausgestaltung nach Anspruch 6 ist dem Patienten zugeordnet über die gerichtete Schallquelle eine individualisierte Kategorie von Informationen/Unterhaltung ausgebbar.

Die Vorteile sind im Zusammenhang mit Anspruch 3 bereits erläutert.

Die mediale Gerätekombination kann im Rahmen der vorliegenden Erfindung außer der Schallquelle eine Basiseinheit aufweisen zur Bereitstellung der Information bzw. Unterhaltung. Diese Basiseinheit kann beispielsweise ein Abspielgerät für Ton- und/oder Bildträger sein oder auch ein Empfangsgerät zum Empfang kabelgebunden übertragbarer Signale bzw. per drahtlos übertragener Signale. Die mediale Gerätekombination kann weiterhin eine Anzeigeeinheit umfassen zur visuellen Darstellung der Information bzw. Unterhaltung. Diese Anzeigeeinheit kann ein Bildschirm oder auch ein Display sein.

Die im Zusammenhang mit der vorliegenden Erfindung angesprochenen Behandlungsplätze können neben der Liege bzw. dem Sitz für den Patienten auch ein Behandlungsgerät aufweisen. Dieses Behandlungsgerät kann beispielsweise ein Blutbehandlungs- oder ein Infusionsgerät umfassen. Bei dem Blutbehandlungsgerät kann es sich insbesondere um ein Hämodialysegerät handeln. Hierbei erweist es sich als besonders vorteilhaft, dass dem Patienten der Aufenthalt erleichtert wird, indem ihm die Zeit, für die er während der medizinischen Behandlung an den Platz gebunden ist, angenehmer gestaltet werden kann.

Ein Ausführungsbeispiel der Erfindung ist in der Zeichnung dargestellt. Es zeigt dabei im einzelnen:
- Fig. 1:: ein erstes Ausführungsbeispiel eines medizinischen Behandlungsraumes mit einer sternförmigen Anordnung der medizinischen Behandlungsplätze und
- Fig. 2:: ein weiteres Ausführungsbeispiel eines medizinischen Behandlungsraumes, bei dem die medizinischen Behandlungsplätze parallel angeordnet sind.

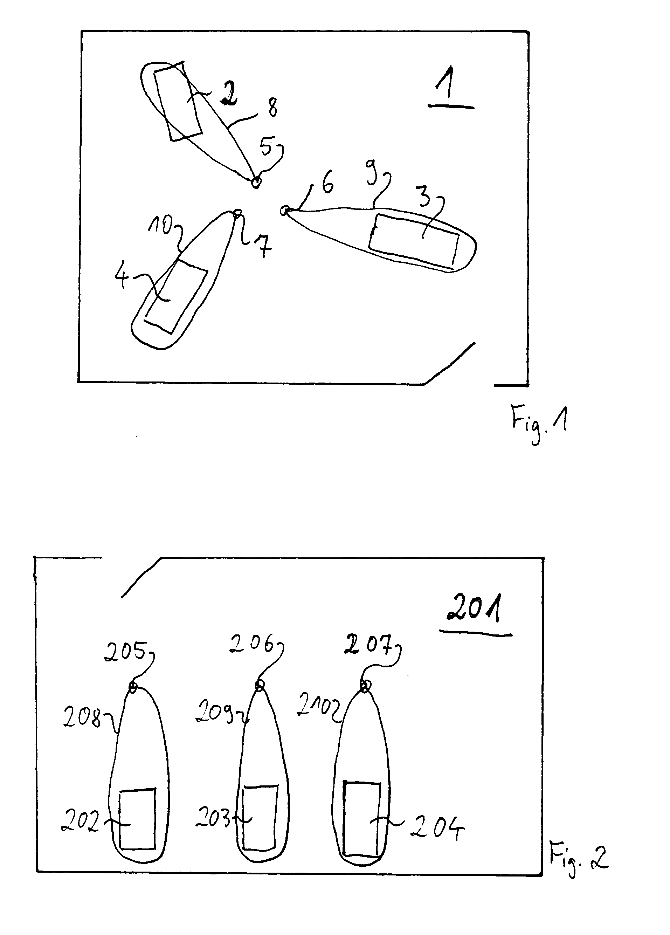

Figur 1 zeigt ein erstes Ausführungsbeispiel eines medizinischen Behandlungsraumes 1 mit einer sternförmigen Anordnung der medizinischen Behandlungsplätze 2, 3, 4.

Den medizinischen Behandlungsplätzen 2, 3, 4 sind jeweils eine Schallquelle 5, 6, 7 zugeordnet, die eine solche Abstrahlcharakteristik 8, 9, 10 haben, dass jedem der medizinischen Behandlungsplätze jeweils einer der Schallkegel zugeordnet ist. Dies wird durch die Begrenzung der "Kegel" der Schallausbreitung in seitlicher Richtung erreicht. Die Schallquellen können beispielsweise an der Decke montiert sein.

Zur Realisierung wird auf den eingangs bereits beschriebenen Stand der Technik verwiesen.

Figur 2 zeigt ein weiteres Ausführungsbeispiel eines medizinischen Behandlungsraumes 201, bei dem die medizinischen Behandlungsplätze 202, 203, 204 parallel und in dem gezeigten Ausführungsbeispiel entlang einer Seitenwand angeordnet sind. Die Schallquellen 205, 206, 207 können hier auf einer Schiene montiert sein, die ebenfalls an der Decke montiert sein kann. Auch hier ist wieder die jeweilige Abstrahlcharakteristik 208, 209, 210 der Schallquellen 205, 206, 207 zu sehen, die sich auf die Behandlungsplätze 202, 203, 204 erstreckt und eine getrennte Wahrnehmung ermöglicht.

## Patentansprüche

1. Verwendung, in einem Behandlungsraum (1, 201), einer gerichteten Schallquelle (5, 6, 7, 205, 206, 207) als Bestandteil einer medialen Gerätekombination in Verbindung mit einem Behandlungsplatz (2, 3, 4, 202, 203, 204) für einen Patienten, an dem sich der Patient für einen mehrere Stunden umfassenden Zeitraum aufhält, wobei mehrere dieser Behandlungsplätze im Behandlungsraum angeordnet sind und wobei die Schallquelle (5, 6, 7, 205, 206, 207) der medialen Gerätekombination jeweils den einzelnen Behandlungsplätzen (2, 3, 4, 202, 203, 204) individuell zugewiesen ist.

2. Verwendung nach Anspruch 1,
**dadurch gekennzeichnet, dass** der Behandlungsplatz (2, 3, 4, 202, 203, 204) ein Dialysebehandlungsplatz ist.

3. Verwendung nach einem der vorhergehenden Ansprüche,
**dadurch gekennzeichnet, dass** dem Patienten zugeordnet über die gerichtete Schallquelle (5, 6, 7, 205, 206, 207) eine individualisierte Kategorie von Informationen/Unterhaltung ausgegeben wird.

4. Medizinischer Behandlungsraum (1, 201), der mehrere medizinische Behandlungsplätze (2, 3, 4, 202, 203, 204) aufweist, die jeweils aus einem Sitz und/oder einer Liege bestehen, auf dem bzw. der sich ein Patient bei einer medizinischen Behandlung für einen mehrere Stunden umfassenden Zeitraum befindet, wobei den medizinischen Behandlungsplätzen (2, 3, 4, 202, 203, 204) eine mediale Gerätkombination mit einer Schallquelle zugeordnet ist, wobei die Schallquelle (5, 6, 7, 205, 206, 207) der medialen Gerätekombination jeweils den einzelnen Behandlungsplätzen (2, 3, 4, 202, 203, 204) individuell zuweisbar ist, **dadurch gekennzeichnet, dass** die Zuweisung der Schallquelle (5, 6, 7, 205, 206, 207) durch wenigstens eine gerichtete Schallquelle (5, 6, 7, 205, 206, 207) erfolgt.

5. Medizinischer Behandlungsraum nach Anspruch 4,
**dadurch gekennzeichnet, dass** die Behandlungsplätze (2, 3, 4, 202, 203, 204) Dialysebehandlungsplätze sind.

6. Medizinischer Behandlungsraum nach Anspruch 4 oder 5,
**dadurch gekennzeichnet, dass** dem Patienten zugeordnet über die gerichtete Schallquelle (5, 6, 7, 205, 206, 207) eine individualisierte Kategorie von Informationen/Unterhaltung ausgebbar ist.

## Claims

1. Use, in a treatment room (1, 201), of a directed sound source (5, 6, 7, 205, 206, 207), which is part of a combination of media devices, in conjunction with a patient treatment station (2, 3, 4, 202, 203, 204) at which the patient spends a period of several hours, a plurality of these treatment stations being set up in the treatment room and the sound source (5, 6, 7, 205, 206, 207) for the combination of media devices being individually assigned to each of the individual treatment stations (2, 3, 4, 202, 203, 204).

2. Use according to claim 1,
**characterised in that** the treatment station (2, 3, 4, 202, 203, 204) is a dialyser station.

3. Use according to any one of the preceding claims,
**characterised in that** the patient is supplied with a personalised category of information/entertainment assigned to him/her via the directed sound source (5, 6, 7, 205, 206, 207).

4. Medical treatment room (1, 201) featuring a plurality of medical treatment stations (2, 3, 4, 202, 203, 204), each of which consists of a seat and/or a bed on which a patient sits or lies for a period of several hours during a medical treatment, a combination of media devices, including a sound source, being assigned to said medical treatment stations (2, 3, 4, 202, 203, 204), said sound source (5, 6, 7, 205, 206, 207) for the combination of media devices being individually assignable to each of the individual treatment stations (2, 3, 4, 202, 203, 204),
**characterised in that** assignment of the sound source (5, 6, 7, 205, 206, 207) is effected by means of at least one directed sound source (5, 6, 7, 205, 206, 207).

5. Medical treatment station according to claim 4,
**characterised in that** the treatment stations (2, 3, 4, 202, 203, 204) are dialyser stations.

6. Medical treatment station according to claim 4 or 5,
**characterised in that** the patient can be supplied with a personalised category of information/entertainment assigned to him/her via the directed sound source (5, 6, 7, 205, 206, 207).

## Revendications

1. Utilisation, dans une salle de traitement (1, 201), d'une source acoustique orientée (5, 6, 7, 205, 206, 207) en tant que composant d'une combinaison d'appareils multimédia en relation avec un emplacement de traitement (2, 3, 4, 202, 203, 204) pour un patient, emplacement sur lequel le patient passe une durée de temps de plusieurs heures, plusieurs de ces emplacements de traitement étant disposés dans la salle de traitement, et la source acoustique (5, 6, 7, 205, 206, 207) de la combinaison d'appareils multimédia étant affectée individuellement à chacun des emplacements de traitement (2, 3, 4, 202, 203, 204).

2. Utilisation selon la revendication 1, **caractérisée en ce que** l'emplacement de traitement (2, 3, 4, 202, 203, 204) est un emplacement de dialyse.

3. Utilisation selon l'une des revendications précédentes, **caractérisée en ce qu'**une catégorie individualisée d'informations / de divertissement est fournie au patient, par la source acoustique orientée.

4. Salle de traitement médical (1, 201) qui présente plusieurs emplacements de traitement médical (2, 3, 4, 202, 203, 204) qui se composent chacun d'un siège et/ou d'un lit sur lequel ou lesquels se trouve le patient lors d'un traitement médical pendant une durée de temps de plusieurs heures, une combinaison d'appareils multimédia avec une source acoustique étant attribuée aux emplacements de traitement médical (2, 3, 4, 202, 203, 204), la source acoustique (5, 6, 7, 205, 206, 207) de la combinaison d'appareils multimédia pouvant être assignée individuellement à chacun des emplacements de traitement (2, 3, 4, 202, 203, 204), **caractérisée en ce que** l'assignation de la source acoustique (5, 6, 7, 205, 206, 207) est réalisée par au moins une source acoustique orientée (5, 6, 7, 205, 206, 207).

5. Salle de traitement selon la revendication 4, **caractérisée en ce que** les emplacements de traitement (2, 3, 4, 202, 203, 204) sont des emplacements de dialyse.

6. Salle de traitement selon la revendication 4 ou 5, **caractérisée en ce qu'**une catégorie individualisée d'informations / de divertissement peut être fournie au patient, par la source acoustique orientée.
